# EUROPÄISCHE PATENTSCHRIFT

(11) **EP 2 431 062 B1**
(45) Veröffentlichungstag und Bekanntmachung des Hinweises auf die Patenterteilung: **03.10.2012**
(21) Anmeldenummer: 11181120.4
(22) Anmeldetag: 13.09.2011
(51) Int. Cl.: A61M 1/00, A61B 18/14

(54) **Medizinisches Instrument zum Saugen und Spülen**
Medical instrument for suction and rinsing
Instrument médical destiné à l'aspiration et au rinçage

(30) Priorität: 17.09.2010 DE 102010045680
(43) Veröffentlichungstag der Anmeldung: 21.03.2012
(73) Patentinhaber: Karl Storz GmbH & Co. KG, 78532 Tuttlingen (DE)
(72) Erfinder: Efinger, Andreas, 78532 Tuttlingen (DE); Pilz, Kevin, 78532 Tuttlingen (DE)

(56) Entgegenhaltungen:
- EP-A1- 1 736 200
- US-A- 5 254 117
- US-A- 5 496 314
- US-B1- 6 231 591
- US-B2- 7 172 572

## Beschreibung

Die Erfindung betrifft ein medizinisches Instrument zum Saugen und Spülen mit einer Handhabe, einem an der Handhabe festlegbaren Saug-/Spülrohr sowie mit einer in der Handhabe angeordneten Ventileinheit mit mindestens einem in einem Saugkanal angeordneten Ventil und mit mindestens einem in einem Spülkanal angeordneten Ventil, wobei jedes Ventil einen Ventilstößel zum Verschließen des zugehörigen Saug- oder Spülkanals aufweist und der Ventilstößel mittels eines mit nur einem Finger betätigbaren Ventilantriebs antreibbar i st.

Derartige medizinische Instrumente zum Saugen und Spülen dienen bei operativen Eingriffen und insbesondere bei endoskopischen Operationen dazu, an der Operationsstelle im Körper des Patienten Spül- und/oder Absaugvorgänge durchzuführen. Bei der endoskopischen Chirurgie ist es beispielsweise erforderlich, das Endoskop-Objektiv mittels einer Spülflüssigkeit von Blut, Sekret oder Beschlag zu reinigen, um einen jederzeit freien Blick auf das Operationsgebiet zu gewährleisten. Der Saugkanal dient dazu, Spülflüssigkeit, Blut oder Sekret aus dem Operationsgebiet zu entfernen.

Aus der DE 196 06 194 C2 ist ein gattungsgemäßes medizinisches Instrument zum Saugen und Spülen bekannt. Dieses mit Tastenventilen ausgestattete medizinische Instrument weist eine mit einem Finger bedienbare Ventileinheit mit jeweils einem Tastenventil für den Saugkanal und den Spülkanal auf, um die Saug- oder Spülleistung im jeweiligen Kanal individuell regulieren zu können. Nachteilig an dieser bekannten Konstruktion ist, dass Tastenventile kaum zu Feindosierung von Fluidströmen geeignet sind, da der mit dem Ventilstößel gekoppelte Drucktaster permanent gedrückt werden muss und ein Halten in einer bestimmten Stellung nur schwer zu gewährleisten ist. Darüber hinaus sind die Ventile fest in den Kanälen angeordnet, wodurch das Reinigen deutlich erschwert wird.

Ein Instrument gemäβ dem Oberbegriff des Anspruchs 1 lehrt die US 5 496 314.

Davon ausgehend liegt der Erfindung die Aufgabe zugrunde, ein medizinisches Instrument zum Saugen und Spülen zu schaffen, das eine möglichst präzise und einfache Regelung der Saug- und Spülleistung gewährleistet.

Die Lösung dieser Aufgabenstellung ist erfindungsgemäß dadurch gekennzeichnet, dass die Ventile als feindosierbare Ventile ausgebildet sind und, dass das mindestens eine Ventil des Saugkanals so ausgebildet ist, dass das Ventil bei Nichtbetätigung in der letzten Ventilstellung verharrt. Das mindestens eine Ventil des Spülkanals ist dagegen so ausgebildet ist, dass das Ventil bei Nichtbetätigung den Spülkanal automatisch schließt.

Durch die Ausbildung der Ventile als Feindosierventile besteht die Möglichkeit, die jeweiligen Fluidströme durch den Saugkanal und den Spülkanal exakt zu dosieren. Zusätzlich sind die Ventile so ausgebildet, dass das Spülkanalventil automatisch schließt, um einen unkontrollierten Spülflüssigkeitsstrom ins Operationsgebiet zu verhindern wenn das Ventil nicht oder nicht mehr betätigt wird. Dahingegen verbleibt das Saugkanalventil bei Nichtbetätigung in der jeweiligen letzten Stellung, um den Operateur zu entlasten.

Die einfache Einhand- und sogar Einfinger-Bedienbarkeit des erfindungsgemäß ausgebildeten medizinischen Instruments kann dadurch vereinfacht werden, dass im Spülkanal und im Saugkanal jeweils nur ein Ventil angeordnet ist und die Ventilantriebe für die beiden Ventile hintereinander in der Ventileinheit angeordnet sind. Alternativ besteht auch die Möglichkeit, Ventilantriebe für die beiden Ventile nebeneinander in der Ventileinheit anzuordnen, was jedoch zu einer breiteren Bauweise der Ventileinheit führt.

Gemäß einer praktischen Ausführungsform zur Ausbildung des Spülkanalventils wird mit der Erfindung vorgeschlagen, dass der Ventilantrieb des im Spülkanal angeordneten Ventils über ein Federelement so vorgespannt ist, dass der Ventilantrieb den Ventilstößel in die den Spülkanal schließende Stellung antreibt.

Gemäß einer bevorzugten Ausführungsform der Erfindung wird vorgeschlagen, dass der Ventilantrieb des im Spülkanal angeordneten Ventils als verschwenkbar gelagerter Kipphebel ausgebildet ist, der über ein Mitnahmeelement mit dem Ventilstößel des Ventils in Wirkverbindung steht, wobei das Mitnahmeelement vorteilhafterweise als am Kipphebel angeformter Nocken ausgebildet ist. Die Ausbildung des einen Ventilantriebs als Kipphebel gewährleistet eine einfache und ermüdungsarme Betätigung dieses Ventils, da das Vor-oder Zurückdrücken eines Kipphebels nur mit einem geringen Kraftaufwand verbunden ist. Durch die Wahl des Lagerpunktes des Kipphebels sowie die Ausgestaltung des Mitnahmeelements lässt sich darüber hinaus das Übersetzungsverhältnis der Kraftübertragung vom Kipphebel auf den Ventilstößel einstellen.

Zur Ausbildung des Saugkanalventils wird gemäß einer bevorzugten Ausführungsform der Erfindung vorgeschlagen, dass der Ventilantrieb des im Saugkanal angeordneten Ventils als Schieber ausgebildet ist, der über ein Mitnahmeelement mit dem Ventilstößel des Ventils in Wirkverbindung steht, wobei das Mitnahmeelement vorteilhafterweise als an der Unterseite des Schiebers angeordnete Platte mit einer vorteilhafterweise als schiefe Ebene ausgebildeten Steuerkurve ausgebildet ist. Die Ausbildung des Ventilantriebs für das Saugkanalventil als Schieber stellt eine besonders einfache und vorteilhafte Ausgestaltung dar, da dieses Ventil bei Nichtbetätigung in der jeweiligen letzten Position verharren soll, was bei einem Schieber ohne großen Aufwand zu verwirklichen ist.

Um zu gewährleisten, dass der Schieber bei Nichtbetätigung in der jeweiligen letzten Position verharrt wird gemäß einer praktischen Ausführungsform der Erfindung vorgeschlagen, dass der Schieber über ein Rastelement in seiner jeweiligen Position fixierbar ist.

Dabei ist das Rastelement in einer Ausführungsform als Kugelraste ausgebildet, die mit an der Unterseite des Schiebers ausgebildeten vorgegebenen Rastpositionen zusammenwirkt. In einer anderen vorteilhaften Ausführungsform ist das Rastelement als Eingriffselement ausgebildet, das mit dem Ventilstößel des Ventils in Wirkverbindung steht und das mit am Mitnahmeelement ausgebildeten Rastpositionen zusammenwirkt. Auf diese Weise kann mit einer geringen Zahl von Bauteilen erreicht werden, dass der Schieber in einer eingestellten Position verharrt, ohne dass der Anwender diesen mit dem Finger fixieren muss. Um das erfindungsgemäße medizinische Instrument und insbesondere die Ventile gründlich reinigen zu können, beispielsweise mittels Autoklavierung, wird mit der Erfindung weiterhin vorgeschlagen, dass die Ventileinheit als Ganzes lösbar in der Handhabe angeordnet ist, das heißt, dass die Ventileinheit zu Reinigungszwecken komplett aus der Handhabe ausbaubar ist.

Die Reinigung der Ventile der Ventileinheit wird erfindungsgemäß dadurch verbessert, dass die Ventilstößel der Ventile ausschließlich bei aus der Handhabe entnommener Ventileinheit in der offenen Stellung arretierbar sind. Die Arretierung in der offenen Stellung stellt sicher, dass die zu reinigenden Ventile vollständig durchströmt werden. Diese Arretierung der Ventilstößel in der offenen Stellung ist aber aus Sicherheitsgründen nur im ausgebauten Zustand der Ventileinheit möglich, um eine Fehlbedienung des medizinischen Instruments auszuschließen.

Schließlich wird mit der Erfindung vorgeschlagen, dass das Saug-/Spülrohr relativ zur Handhabe verdrehbar ist, um insbesondere bei gebogen ausgebildeten Saug-/Spülrohren eine exakte Ausrichtung des Instruments bei gleichzeitig einfacher Handhabung für den Operateur zu ermöglichen.

Weitere Merkmale und Vorteile der Erfindung ergeben sich anhand der zugehörigen Zeichnungen, in denen zwei Ausführungsbeispiele eines erfindungsgemäßen medizinischen Instruments zum Saugen und Spülen nur beispielhaft dargestellt sind, ohne die Erfindung auf diese Ausführungsbeispiele zu beschränken. In den Zeichnungen ist zeigt:
- Fig. 1: eine perspektivische Seitenansicht eines erfindungsgemäßen medizinischen Instruments zum Saugen und Spülen;
- Fig. 2: einen Längsschnitt durch die Handhabe des medizinischen Instruments gemäß Fig. 1;
- Fig. 3: eine vergrößerte Ansicht der Ventileinheit gemäß Fig. 2;
- Fig. 4: eine Ansicht gemäß Fig. 3, jedoch eine zweite Ausführungsform einer Ventileinheit darstellend und
- Fig. 5: eine Ansicht einer weiteren Ausführungsform der Ventileinheit.

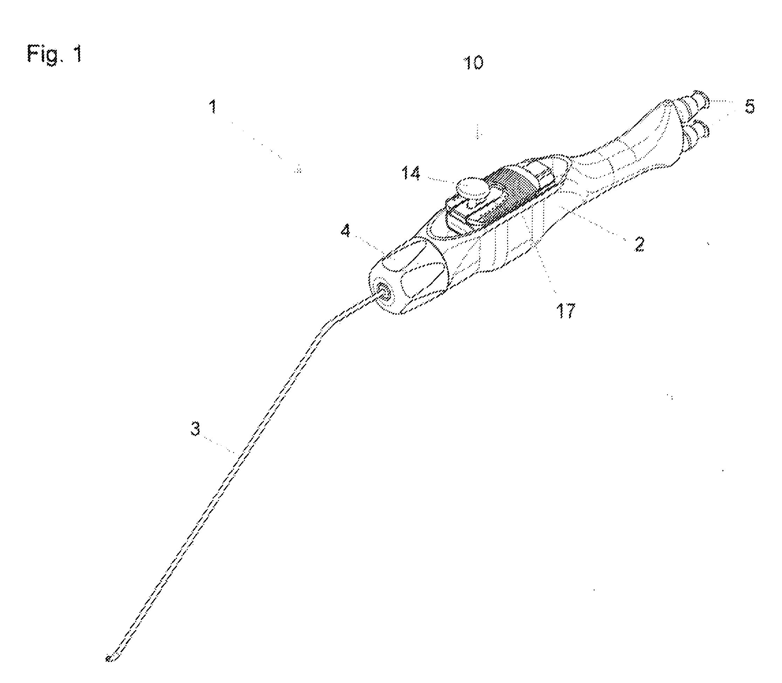

Bei dem in Fig. 1 dargestellten medizinischen Instrument 1 handelt es sich um ein Saug-und Spülinstrument, wie dies insbesondere bei endoskopischen Operationen dazu dient, an der Operationsstelle im Körper des Patienten Spül- und/oder Absaugvorgänge durchzuführen, um einerseits beispielsweise das Endoskop-Objektiv mittels einer Spülflüssigkeit von Blut, Sekret oder Beschlag zu reinigen, um einen jederzeit freien Blick auf das Operationsgebiet zu gewährleisten und, um andererseits die Spülflüssigkeit, Blut oder Sekret aus dem Operationsgebiet zu entfernen.

Das in Fig. 1 dargestellte medizinische Instrument 1 zum Saugen und Spülen besteht im Wesentlichen aus einer Handhabe 2, an deren distalem Ende ein Saug-/Spülrohr 3 mittels einer Überwurfmutter 4 festlegbar ist. Um insbesondere bei gebogen ausgebildeten Saug-/Spülrohren 3 eine exakte Ausrichtung des medizinischen Instruments 1 bei gleichzeitig einfacher Handhabung für den Operateur zu ermöglichen, ist das Saug-/Spülrohr 3 relativ zur Handhabe 2 verdrehbar.

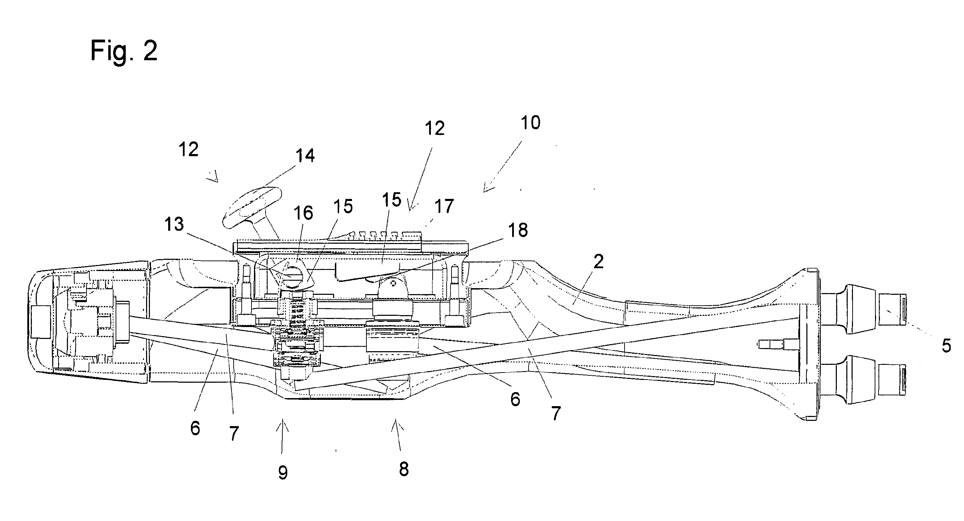

Am proximalen Ende weist die Handhabe 2 zwei Schlauchanschlussstutzen 5 zum Verbinden der Handhabe 2 mit einer externen Saug- und Spülquelle auf. Von den Schlauchanschlussstutzen 5 erstrecken sich ein Saugkanal 6 und ein Spülkanal 7 bis in das Saug-/Spülrohr 3, wie dies der Abbildung Fig. 2 zu entnehmen ist.

Zum Dosieren des durch den Saugkanal 6 und den Spülkanal 7 strömenden Fluidstroms ist in jedem der Kanälen 6 und 7 jeweils ein Ventil 8 und 9 angeordnet. Beide Ventile 8 und 9 sind in einer Ventileinheit 10 angeordnet, die lösbar in der Handhabe 2 festgelegt ist. Das Festlegen der Ventileinheit 10 in der Handhabe 2 kann beispielsweise über eine Rastverbindung erfolgen.

Wie insbesondere aus Fig. 1 ersichtlich, ist die Handhabe 2 so aufgebaut, dass die Handhabe 2 im Einhandbetrieb bedienbar ist. Um die Handhabung weiter zu vereinfachen, ist die Ventileinheit 10 so ausgebildet, dass die Ventile 8 und 9 mit nur einem Finger der Haltehand, beispielsweise dem Zeigefinger, bedienbar sind. Selbstverständlich ist es alternativ auch möglich, die Ventile 8 und 9 nebeneinander in der Ventileinheit 10 anzuordnen.

Der Aufbau der Ventileinheit 10 sowie der Ventile 8 und 9 ist den Abbildungen Fig. 2 bis 4 zu entnehmen.

Jedes Ventil 8 und 9 weist einen Ventilstößel 11 zum Schließen und wieder Freigeben des zugehörigen Saugkanals 6 oder Spülkanals 7 sowie einen manuell bedienbaren Ventilantrieb 12 auf, über den der Ventilstößel zwischen den beiden Endstellungen, nämlich offener Kanal und verschlossener Kanal, stufenlos verstellbar ist.

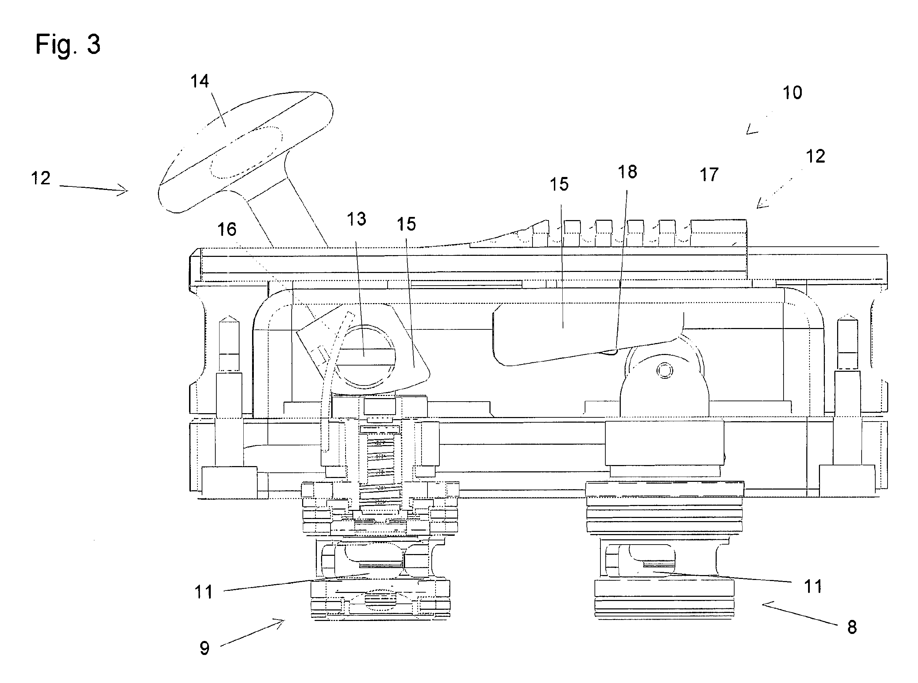

Fig. 3 zeigt eine erste Ausführungsform zur Ausgestaltung der Ventileinheit 10.

Bei der dargestellten Ausführungsform ist der Ventilantrieb 12 als um einen Drehpunkt 13 verschwenkbarer Kipphebel 14 ausgebildet, der über ein als angeformter Nocken ausgebildetes Mitnahmeelement 15 mit dem Ventilstößel 11 in Wirkverbindung steht. Um zu gewährleisten, dass über den Spülkanal 7 nicht unkontrolliert Spülflüssigkeit ins Operationsgebiet geleitet werden kann, ist das Ventil 9 über ein als Blattfeder ausgebildetes Federelement 16 in die den Spülkanal 7 verschließende Stellung so vorgespannt, dass das Ventil 9 den Spülkanal 7 bei Nichtbetätigung des Ventilantriebs 12 automatisch verschließt. Durch die Wahl des Drehpunktes 13 des Kipphebels 14 sowie die Ausgestaltung des Mitnahmeelements 15 lässt sich das Übersetzungsverhältnis der Kraftübertragung vom Kipphebel 14 auf den Ventilstößel 11 einstellen. Das Federelement 16 kann auch als Spiralfeder oder anderes bekanntes Federelement ausgebildet sein kann.

Um die Einhandbedienung und insbesondere die Feindosierung zu erleichtern, ist der Kipphebel 14 über das Federelement 16 in der nicht betätigten Position nach distal gekippt vorgespannt. Bei Betätigung kann der Kipphebel 14 mit einem Finger einer Hand des Operateurs gegen die Kraft des Federelements 16 bewegt und der Spülkanal 7 nach Bedarf freigegeben werden.

Alternativ zu der dargestellten Ausgestaltungsform des Ventilantriebs 12 als Kipphebel 14 ist es selbstverständlich auch möglich, den Ventilantrieb 12 beispielsweise als Schieber mit einer Schneckenwelle auszugestalten oder aber das Mitnahmeelement 15 als Pleulstange oder schiefe Ebene auszugestalten. Wichtig ist aber immer, dass das Ventil 9 eine Feindosierung des Spülflüssigkeitsstroms durch den Spülkanal 7 ermöglicht und, dass das Ventil 9 bei Nichtbetätigung des Ventilantriebs 12 automatisch in die den Spülkanal 7 verschließende Stellung übergeht.

Bei der in Fig. 3 dargestellten Ausführungsform ist der Ventilantrieb 12 des im Saugkanal 6 anzuordnenden Ventils 8 als in Längsrichtung der Handhabe 2 verstellbarer Schieber 17 ausgebildet, der über ein an der Unterseite des Schiebers 17 angeordnetes Mitnahmeelement 15 mit dem Ventilstößel 11 in Wirkverbindung steht. Um ein stufenloses Öffnen und Schließen des Saugkanals 6 zu ermöglichen, ist die dem Ventilstößel 11 zugewandte Seite des Mitnahmeelements 11 als schiefe Ebene 18 ausgebildet.

Der Ventilantrieb 12 des Ventils 8 ist so ausgebildet, dass das Ventil 8 bei Nichtbetätigung des Ventilantriebs 12 unverändert in der letzten Stellung verbleibt, so dass der Operateur bei einmal eingestellter Saugleistung den Ventilantrieb 12 des Ventils 8 nicht mehr betätigen muss.

Alternativ zur Ausbildung der dem Ventilstößel 11 zugewandten Seite des Mitnahmeelements 11 als schiefe Ebene 18 ist es auch möglich anders geformte Steuerkurven vorzusehen, sofern diese eine Feindosierung erlauben und bei Nichtbetätigung des Ventilantriebs 12 unverändert in der letzten Stellung verbleiben.

Auch ist es alternativ zu der dargestellten Ausgestaltungsform des Ventilantriebs 12 als Schieber 17 möglich, den Ventilantrieb 12 beispielsweise als Hebel auszugestalten oder aber das Mitnahmeelement 15 als Pleulstange oder Schneckenwelle auszugestalten. Wichtig ist aber immer, dass das Ventil 8 eine Feindosierung der Saugleistung durch den Saugkanal 8 ermöglicht und, dass das Ventil 8 bei Nichtbetätigung des Ventilantriebs 12 in der letzten Stellung verbleibt.

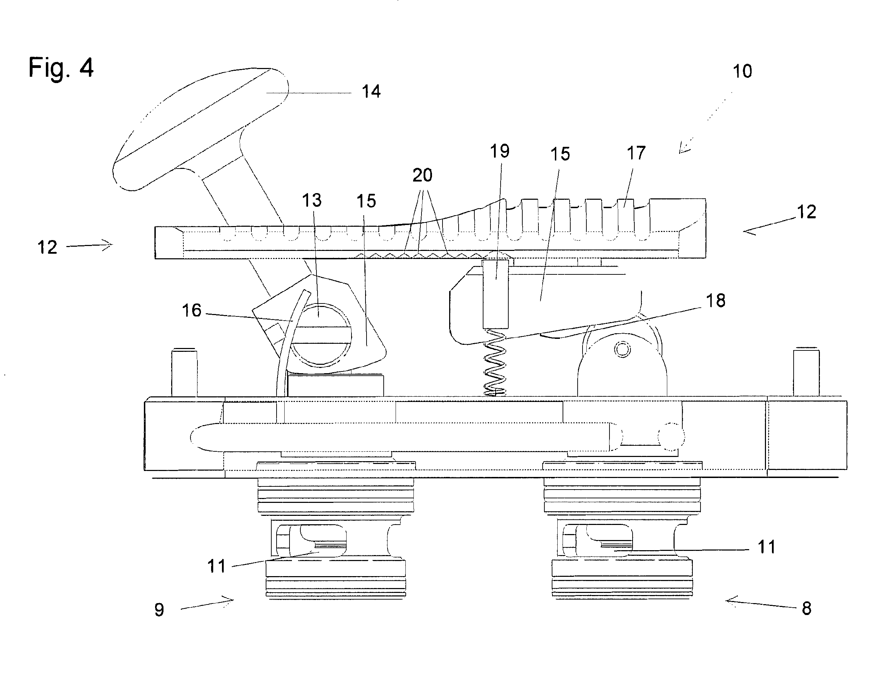

Fig. 4 zeigt eine zweite Ausführungsform zur Ausgestaltung der Ventileinheit 10. Von der in Fig. 3 dargestellten und voranstehend beschriebenen ersten Ausführungsform unterscheidet sich diese Ventileinheit 10 durch die Ausbildung des Ventilantriebs 12 für das im Saugkanal 6 anzuordnende Ventil 8.

Um das Ventil 8 bei Nichtbetätigung des Ventilantriebs 12 unverändert in der letzten Stellung zu halten, ist unterhalb des Schiebers 17 eine federbelastete Kugelraste 19 angeordnet, die in an der Unterseite des Schiebers 17 ausgebildete Rastpositionen 20 fixierend eingreifen kann. Ansonsten entspricht der Ventilantrieb 12 für das im Saugkanal 6 anzuordnende Ventil 8 im Aufbau und in der Arbeitsweise dem zuvor beschriebenen und in Fig. 3 dargestellten Ventilantrieb 12.

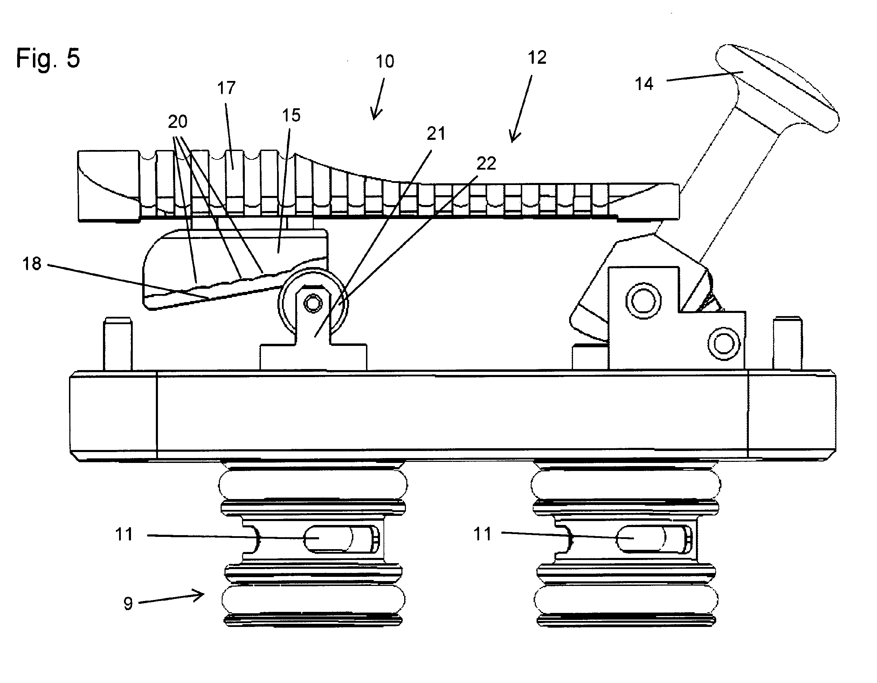

Fig. 5 zeigt eine dritte Ausführungsform der Ventileinheit 10. Von der in Fig. 4 dargestellten Ausführungsform unterscheidet sich diese Ventileinheit 10 durch eine andere Ausgestaltung des Rastelements. Es ist ein Eingriffselement 21 vorgesehen, das mit dem Ventilstößel 11 in Wirkverbindung steht. Das Eingriffselement 21 greift an seinem dem Schieber 17 zugewandten Ende in Rastpositionen 20 an der schiefen Ebene 18 ein. In dieser Darstellung ist das dem Schieber 17 zugewandte Ende des Eingriffselements 21 zur Verbesserung der Handhabung mit einem Rad 22 versehen, das bei Betätigung des Schiebers 17 von einer Rastposition 20 in die nächste rollt und bei Loslassen des Schiebers 17 in der eingestellten Rastposition 20 verharrt und so den Schieber 17 fixiert.

Wie aus Fig. 3 und 4 ersichtlich, ist die Ventileinheit 10, beispielsweise zu Reinigungszwecken, als Ganzes aus der Handhabe 2 entnehmbar. In dieser aus der Handhabe 2 entnommenen Stellung der Ventileinheit 10 sind die Ventilstößel 11 der Ventile 8 und 9 in der offenen Stellung arretierbar, um eine vollständige Reinigung der Ventile 8 und 9, beispielsweise mittels Autoklavieren, zu ermöglichen. Diese Arretierung der Ventilstößel 11 in der offenen Stellung ist zur Verbesserung der Sicherheit nur im ausgebauten Zustand der Ventileinheit 10 möglich, um eine Fehlbedienung des medizinischen Instruments 1 auszuschließen.

Ein wie zuvor beschrieben ausgebildetes medizinisches Instrument 1 zeichnet sich dadurch aus, dass einerseits eine präzise Regelung der Saug- und Spülleistung durch die Ausbildung der Ventile 8 und 9 als Feindosierventile gewährleistet und andererseits das gesamte Instrument 1 und insbesondere die Ventileinheit 10 einfach und gründlich zu reinigen ist.

Die Anordnung und Ausbildung der Ventilantriebe 12 in der Ventileinheit 10 ermöglicht es, dass das medizinische Instrument 1 besonders ruhig gehalten werden kann und auch bei der Bedienung der Ventilantriebe 12 nicht kippt, was ein präzises Arbeiten, insbesondere in der Neurochirurgie, ermöglicht.

Neben der Möglichkeit, die Ventileinheit 10 aus der Handhabe 2 ausbauen zu können, wird die einfach und gute Reinigbarkeit dadurch ermöglicht, dass die Handhabe 2 als kompakte geschlossenen Baueinheit ausgebildet ist.

### Bezugszeichenliste

- 1: medizinisches Instrument
- 2: Handhabe
- 3: Saug-/Spülrohr
- 4: Überwurfmutter
- 5: Schlauchanschlussstutzen
- 6: Saugkanal
- 7: Spülkanal
- 8: Ventil (Saugkanal)
- 9: Ventil (Spülkanal)
- 10: Ventileinheit
- 11: Ventilstößel
- 12: Ventilantrieb
- 13: Drehpunkt
- 14: Kipphebel
- 15: Mitnahmeelement
- 16: Federelement
- 17: Schieber

- 18: schiefe Ebene
- 19: Kugelraste
- 20: Rastpositionen
- 21: Eingriffselement
- 22: Rad

## Patentansprüche

1. Medizinisches Instrument zum Saugen und Spülen mit einer Handhabe (2), einem an der Handhabe (2) festlegbaren Saug-/Spülrohr (3) sowie mit einer in der Handhabe (2) angeordneten Ventileinheit (10) mit mindestens einem in einem Saugkanal (6) angeordneten Ventil (8) und mit mindestens einem in einem Spülkanal (7) angeordneten Ventil (9), wobei jedes Ventil (8, 9) einen Ventilstößel (11) zum Verschließen des zugehörigen Saug- oder Spülkanals (6, 7) aufweist und der Ventilstößel (11) mittels eines mit nur einem Finger betätigbaren Ventilantriebs (12) antreibbar ist, wobei das mindestens eine Ventil (9) des Spülkanals (7) so ausgebildet ist, dass das Ventil (9) bei Nichtbetätigung den Spülkanal (7) automatisch schließt,
**dadurch gekennzeichnet,**
**dass** die Ventile (8, 9) als feindosierbare Ventile ausgebildet sind und dass das mindestens eine Ventil (8) des Saugkanals (6) so ausgebildet ist, dass das Ventil (8) bei Nichtbetätigung in der letzten Ventilstellung verharrt.

2. Medizinisches Instrument nach Anspruch 1, **dadurch gekennzeichnet, dass** im Spülkanal (7) und im Saugkanal (6) jeweils ein Ventil (8, 9) angeordnet ist und die Ventilantriebe (12) für die beiden Ventile (8, 9) hintereinander in der Ventileinheit (10) angeordnet sind.

3. Medizinisches Instrument nach Anspruch 1 oder 2, **dadurch gekennzeichnet, dass** der Ventilantrieb (12) des im Spülkanal (7) angeordneten Ventils (9) über ein Federelement (16) so vorgespannt ist, dass der Ventilantrieb (12) den Ventilstößel (11) in die den Spülkanal (7) schließende Stellung antreibt.

4. Medizinisches Instrument nach Anspruch 3, **dadurch gekennzeichnet, dass** der Ventilantrieb (12) des im Spülkanal (7) angeordneten Ventils (9) als verschwenkbar gelagerter Kipphebel (14) ausgebildet ist, der über ein Mitnahmeelement (15) mit dem Ventilstößel (11) des Ventils (9) in Wirkverbindung steht.

5. Medizinisches Instrument nach Anspruch 4, **dadurch gekennzeichnet, dass** das Mitnahmeelement (15) als am Kipphebel (14) angeformter Nocken ausgebildet ist.

6. Medizinisches Instrument nach Anspruch 1 oder 2, **dadurch gekennzeichnet, dass** der Ventilantrieb (12) des im Saugkanal (6) angeordneten Ventils (8) als Schieber (17) ausgebildet ist, der über ein Mitnahmeelement (15) mit dem Ventilstößel (11) des Ventils (8) in Wirkverbindung steht.

7. Medizinisches Instrument nach Anspruch 6, **dadurch gekennzeichnet, dass** das Mitnahmeelement (15) als an der Unterseite des Schiebers (17) angeordnete Platte mit einer, insbesondere in der Form einer schiefen Ebene (18), ausgebildeten Steuerkurve ausgebildet ist.

8. Medizinisches Instrument nach Anspruch 6 oder 7, **dadurch gekennzeichnet, dass** der Schieber (17) über ein Rastelement in seiner jeweiligen Position fixierbar ist.

9. Medizinisches Instrument nach Anspruche 8, **dadurch gekennzeichnet, dass** das Rastelement als Kugelraste (19) ausgebildet ist, die mit an der Unterseite des Schiebers (17) ausgebildeten vorgegebenen Rastpositionen (20) zusammenwirkt.

10. Medizinisches Instrument nach Anspruch 8, **dadurch gekennzeichnet, dass** das Rastelement als Eingriffselement (21) ausgebildet ist, das mit dem Ventilstößel (11) in Wirkverbindung steht und das mit am Mitnahmeelement (15) ausgebildeten Rastpositionen (20) zusammenwirkt.

11. Medizinisches Instrument nach einem der Ansprüche 1 bis 10, **dadurch gekennzeichnet, dass** die Ventileinheit (10) als Ganzes lösbar in der Handhabe (2) angeordnet ist.

12. Medizinisches Instrument nach Anspruch 11, **dadurch gekennzeichnet, dass** die Ventilstößel (11) der Ventile (8, 9) ausschließlich bei aus der Handhabe (2) entnommener Ventileinheit (10) in der offenen Stellung arretierbar sind.

13. Medizinisches Instrument nach einem der Ansprüche 1 bis 12, **dadurch gekennzeichnet, dass** das Saug-/Spülrohr (3) relativ zur Handhabe (2) verdrehbar ist.

## Claims

1. Medical instrument for suction and irrigation, with a handle (2), with a suction/irrigation pipe (3) that can be fastened to the handle (2), and with a valve unit (10) which is arranged in the handle (2) and which has at least one valve (8) arranged in a suction channel (6) and at least one valve (9) arranged in an irrigation channel (7), each valve (8, 9) having a valve plunger (11) for closing the associated suction or irrigation channel (6, 7), and the valve plunger (11) being able to be driven by means of a valve drive (12), which can be actuated with just one finger, the at least one valve (9) of the irrigation channel (7) being designed such that the valve (9), when not actuated, automatically closes the irrigation channel (7), **characterized in that** the valves (8, 9) are designed as finely adjustable valves, and **in that** the at least one valve (8) of the suction channel (6) is designed such that the valve (8), when not actuated, remains in the last valve position.

2. Medical instrument according to Claim 1, **characterized in that** a valve (8, 9) is arranged respectively in the irrigation channel (7) and in the suction channel (6), and the valve drives (12) for the two valves (8, 9) are arranged one behind the other in the valve unit (10).

3. Medical instrument according to Claim 1 or 2, **characterized in that** the valve drive (12) of the valve (9) arranged in the irrigation channel (7) is pretensioned via a spring element (16) in such a way that the valve drive (12) drives the valve plunger (11) into the position closing the irrigation channel (7).

4. Medical instrument according to Claim 3, **characterized in that** the valve drive (12) of the valve (9) arranged in the irrigation channel (7) is designed as a pivotably mounted rocker arm (14), which is operatively connected to the valve plunger (11) of the valve (9) via a driving element (15).

5. Medical instrument according to Claim 4, **characterized in that** the driving element (15) is designed as a cam formed integrally on the rocker arm (14).

6. Medical instrument according to Claim 1 or 2, **characterized in that** the valve drive (12) of the valve (8) arranged in the suction channel (6) is designed as a slide (17), which is operatively connected to the valve plunger (11) of the valve (8) via a driving element (15).

7. Medical instrument according to Claim 6, **characterized in that** the driving element (15) is designed as a plate arranged on the underside of the slide (17), with a control cam designed in particular in the form of a slanting plane (18).

8. Medical instrument according to Claim 6 or 7, **characterized in that** the slide (17) can be fixed in its respective position by a latching element.

9. Medical instrument according to Claim 8, **characterized in that** the latching element is designed as a ball latch (19), which engages with predefined latching positions (20) formed on the underside of the slide (17).

10. Medical instrument according to Claim 8, **characterized in that** the latching element is designed as an engaging element (21), which is operatively connected to the valve plunger (11) and which engages with latching positions (20) formed on the driving element (15).

11. Medical instrument according to one of Claims 1 to 10, **characterized in that** the valve unit (10) as a whole is arranged releasably in the handle (2).

12. Medical instrument according to Claim 11, **characterized in that** the valve plungers (11) of the valves (8, 9) can be locked in the open position only when the valve unit (10) is removed from the handle (2).

13. Medical instrument according to one of Claims 1 to 12, **characterized in that** the suction/irrigation pipe (3) is rotatable relative to the handle (2).

## Revendications

1. Instrument médical destiné à l'aspiration et au rinçage, comprenant un manche (2), un tube d'aspiration/rinçage (3) pouvant être fixé au manche (2) ainsi qu'une unité de soupape (10) disposée dans le manche (2), avec au moins une soupape (8) disposée dans un canal d'aspiration (6), et avec au moins une soupape (9) disposée dans un canal de rinçage (7), chaque soupape (8, 9) présentant un poussoir de soupape (11) pour fermer le canal d'aspiration ou de rinçage associé (6, 7), et le poussoir de soupape (11) pouvant être entraîné au moyen d'un entraînement de soupape (12) actionnable d'un seul doigt, l'au moins une soupape (9) du canal de rinçage (7) étant réalisée de telle sorte que la soupape (9) ferme automatiquement le canal de rinçage (7) lorsqu'elle n'est pas actionnée,
**caractérisé en ce que**
les soupapes (8, 9) sont réalisées sous forme de soupapes à dosage de précision et **en ce que** l'au moins une soupape (8) du canal d'aspiration (6) est réalisée de telle sorte que la soupape (8) demeure dans la dernière position de soupape lorsqu'elle n'est pas actionnée.

2. Instrument médical selon la revendication 1, **caractérisé en ce que** dans le canal de rinçage (7) et dans le canal d'aspiration (6) est à chaque fois disposée une soupape (8, 9), et les entraînements de soupape (12) pour les deux soupapes (8, 9) sont disposés l'un derrière l'autre dans l'unité de soupape (10).

3. Instrument médical selon la revendication 1 ou 2, **caractérisé en ce que** l'entraînement de soupape (12) de la soupape (9) disposée dans le canal de rinçage (7) est précontraint par le biais d'un élément de ressort (16) de telle sorte que l'entraînement de soupape (12) entraîne le poussoir de soupape (11) dans la position fermant le canal de rinçage (7).

4. Instrument médical selon la revendication 3, **caractérisé en ce que** l'entraînement de soupape (12) de la soupape (9) disposée dans le canal de rinçage (7) est réalisé sous forme de levier basculant (14) monté pivotant, qui est en liaison fonctionnelle avec le poussoir de soupape (11) de la soupape (9) par le biais d'un élément d'entraînement (15).

5. Instrument médical selon la revendication 4, **caractérisé en ce que** l'élément d'entraînement (15) est réalisé sous forme de came façonnée sur le levier basculant (14).

6. Instrument médical selon la revendication 1 ou 2, **caractérisé en ce que** l'entraînement de soupape (12) de la soupape (8) disposée dans le canal d'aspiration (6) est réalisé sous forme de coulisseau (17) qui est en liaison fonctionnelle par le biais d'un élément d'entraînement (15) avec le poussoir de soupape (11) de la soupape (8).

7. Instrument médical selon la revendication 6, **caractérisé en ce que** l'élément d'entraînement (15) est réalisé sous forme de plaque disposée sur le côté inférieur du coulisseau (17), avec une courbe de commande réalisée notamment sous forme de plan oblique (18).

8. Instrument médical selon la revendication 6 ou 7, **caractérisé en ce que** le coulisseau (17) peut être fixé par le biais d'un élément d'encliquetage dans sa position respective.

9. Instrument médical selon la revendication 8, **caractérisé en ce que** l'élément d'encliquetage est réalisé sous forme de verrou à bille (19) qui coopère avec des positions d'encliquetage (20) prédéfinies réalisées sur le côté inférieur du coulisseau (17).

10. Instrument médical selon la revendication 8, **caractérisé en ce que** l'élément d'encliquetage est réalisé sous forme d'élément d'engagement (21) qui est en liaison fonctionnelle avec le poussoir de soupape (11) et qui coopère avec des positions d'encliquetage (20) réalisées sur l'élément d'entraînement (15).

11. Instrument médical selon l'une quelconque des revendications 1 à 10, **caractérisé en ce que** l'unité de soupape (10) est disposée dans son ensemble de manière amovible dans le manche (2).

12. Instrument médical selon la revendication 11, **caractérisé en ce que** les poussoirs de soupape (11) des soupapes (8, 9) peuvent être bloqués dans la position ouverte exclusivement lorsque l'unité de soupape (10) est retirée du manche (2).

13. Instrument médical selon l'une quelconque des revendications 1 à 12, **caractérisé en ce que** le tube d'aspiration/rinçage (3) peut tourner par rapport au manche (2).
